(19) Europäisches Patentamt
European Patent Office
Office européen des brevets

(11) **EP 1 608 261 B1**

(12) **EUROPÄISCHE PATENTSCHRIFT**

(45) Veröffentlichungstag und Bekanntmachung des Hinweises auf die Patenterteilung:
**02.11.2006 Patentblatt 2006/44**

(21) Anmeldenummer: **04724941.2**

(22) Anmeldetag: **01.04.2004**

(51) Int Cl.:
***A61B 5/00*** *(2006.01)*

(86) Internationale Anmeldenummer:
**PCT/AT2004/000117**

(87) Internationale Veröffentlichungsnummer:
**WO 2004/086963 (14.10.2004 Gazette 2004/42)**

(54) **VORRICHTUNG ZUR KONTINUIERLICHEN, NICHT-INVASIVEN MESSUNG DES BLUTDRUCKES**

DEVICE FOR THE CONTINUOUS NON-INVASIVE MEASUREMENT OF BLOOD PRESSURE

DISPOSITIF POUR MESURE NON INVASIVE CONTINUE DE LA TENSION ARTERIELLE

(84) Benannte Vertragsstaaten:
**AT BE BG CH CY CZ DE DK EE ES FI FR GB GR HU IE IT LI LU MC NL PL PT RO SE SI SK TR**

(30) Priorität: **01.04.2003 AT 5092003**

(43) Veröffentlichungstag der Anmeldung:
**28.12.2005 Patentblatt 2005/52**

(73) Patentinhaber: **CNSystems Medizintechnik GmbH 8020 Graz (AT)**

(72) Erfinder:
• **SKRABAL, Falko**
  **8043 GRAZ (AT)**
• **FORTIN, Jürgen**
  **8020 GRAZ (AT)**

(74) Vertreter: **Babeluk, Michael**
**Patentanwalt,**
**Mariahilfer Gürtel 39/17**
**1150 Wien (AT)**

(56) Entgegenhaltungen:
**EP-A- 1 125 546     AT-B- 408 066**
**DE-A- 3 632 592     DE-A- 3 829 456**

• **PENAZ J ET AL: "VIBRATION PLETHYSMOGRAPHY: A METHOD FOR STUDYING THE VISCO-ELASTIC PROPERTIES OF FINGER ARTERIES" MEDICAL AND BIOLOGICAL ENGINEERING AND COMPUTING, PETER PEREGRINUS LTD. STEVENAGE, GB, Bd. 35, Nr. 6, 1. November 1997 (1997-11-01), Seiten 633-637, XP000723476 ISSN: 0140-0118**

EP 1 608 261 B1

**Beschreibung**

Die Erfindung betrifft eine Vorrichtung zur kontinuierlichen, nicht-Invasiven Messung des Blutdruckes nach dem Prinzip der entspannten Arterienwand, wobei an zumindest einem ersten und einem zweiten jeweils eine Arterie vergleichbarer oder identer Dimension enthaltenden Körperteil oder Körperbereich eine erste und eine zweite Druckmanschette vergleichbarer oder identer Dimension mit einer ersten uns einer zweiten aufblasbaren Druckmesskammer angeordnet werden, wobei der Druck in der ersten Druckmesskammer in Abhängigkeit des Messsignals einer plethysmographischen Sensoreinrichtung derart geregelt wird, dass die Amplitude des plethysmographischen Messsignals minimiert wird und wobei in der ersten Druckmesskammer ein Druckmesssignal gewonnen wird.

In der Medizin besteht häufig die Notwendigkeit den Blutdruck von Lebewesen kontinuierlich zu messen. Besonders vorteilhaft ist dabei die nicht-invasive Messung ohne blutige Punktion von Arterien. Eine nicht-invasive Technik, die sich besonders bewährt hat, ist die sogenannte "vascular unloading technique" (auch Prinzip der entspannten Arterlenwand genannt), bei der das sich mit dem Herzschlag pulsatil ändernde Blutvolumen oder der Blutfluss in einer Extremität z.B. im Finger, plethysmographisch, z.B. mittels einer optischen Sensoreinrichtung bestehend Lichtemitter und Lichtdetektor, gemessen wird.

In der Folge wird bei diesem Verfahren über eine Rückkopplungsschleife der Druck In einer außen auf der Arterie aufgebrachten Druckkammer einer aufblasbaren Manschette, die mit ihrem regelbaren Druck die darunter liegenden Arterie beaufschlagt, in Abhängigkeit des plethysmographischen Signals so verändert, dass das sich vorher pulsatil ändernde Volumen bzw. der Blutfluss nun konstant bleibt oder die pulsatilen Veränderungen minimiert werden. Dazu wird während der Systole, in der das Volumen oder der Blutfluss größer ist, der Druck in der Manschette erhöht und in der Diastole, während der das Volumen oder der Blutfluss kleiner ist, der Druck in der Manschette verringert. Damit wird die Arterlenwand völlig entspannt, und in der Arterie und in der Manschette, die jetzt faktisch nur durch eine flottierende Membran (= entspannte Arterienwand) getrennt sind, herrschen gleiche Drucke wie in kommunizierenden Gefäßen, sodass der sogenannte transmurale Druck $P_{Tm}$ gleich Null ist. Der in der Messkammer gemessene Druck $P_M$ entspricht somit gemäß der Gleichung:

$$P_M = P_{Bl} - P_{Tm}$$

mit $P_{Tm} = 0$ direkt dem in der Pulskurve wiedergegebenen arteriellen Blutdruck $P_{Bl}$. Bei der "vascular unloading technique" spricht man von einer closed-loop Messung, wenn der Manschettendruck vom plethysmographischen Signal gesteuert wird (geschlossene Rückkopplungsschleife) und von einer open loop Messung, wenn der Manschettendruck konstant bzw. unabhängig vom plethysmographischen Signal geregelt wird.

Beschrieben wird die "vascular unloading technique" beispielsweise in D1: J. PENAZ: Photoelectric Measaurement of Blood Pressure, Volume and Flow in the finger, Digest of the 10th International Conference on Medical and Biological Engineering 1973 Dresden; D2: G.P. MOLHOEK, K.H. WESSELING, J.J.M. SETTELS, E. VAN VOLLENHOVEN, H. WEEDA, B. DE WIT, A.C. ARNTZENIUS: Evaluation of the Penaz servo-plethysmo-manometer for the continuous, non-invasive measurement of finger blood pressure; Basic Res Cardiool 79: 598-609, 1984 und D3: J.C. DORLAS, J.A. NIJBOER, W.T. BUTJIN, M.A. VAN DER HOEVEN, J.J. SETTELS, K.H. WESSELING: Effects of Peripheral Vasoconstriction on the Blood Pressure in the Finger, Measured Continuously by a New Noninvasive Method (The Finapres®); Anesthesiology 62: 342-345, 1985.

Um den optimalen Anpressdruck der Druckkammer vor der eigentlichen Messung zu ermitteln, ist es bekannt, mittels Druckänderungen, z.B. in Form einer Drucktreppe oder Druckrampe, und vor der Schließung der Rückkopplungsschleife, einen sogenannte "Setpoint" (Arbeitspunkt) zu ermitteln. Es wird dabei in etwa jener Druck der Messkammer als Setpoint gewählt, bei dem die Amplitude des piethysmographischen Signals (z.B. Signal des Lichtdetektors) beim oder zumindest nahe beim Maximum liegt (siehe z.B. D1, D3).

In diesem Zusammenhang ist aus der WO 00/59369 A2 bzw. der AT 408.066 B ein kontinuierliches, nicht-invasives Blutdruckmessgerät bekannt geworden, welches nach dem Prinzip der entspannten Arterienwand arbeitet und eine Doppelfingermanschette an benachbarten Fingern aufweist. Die Druckkammern in beiden Manschetten werden in Abhängigkeit des Messsignals einer plethysmographischen Sensoreinrichtung, jeweils bestehend z.B. aus Lichtemitter und Lichtdetektor, geregelt, wobei beide Druckkammern an eine gemeinsame Druckregelkammer angeschlossen sind. Mit Hilfe eines Umschaltventils kann die Druckbeaufschlagung von einer Manschette auf die andere Manschette umgeschaltet werden. Die gemeinsame Druckregelkammer ist mit einem separaten Einlass- und Auslassventil ausgestattet, wodurch der Druckverlauf In der Druckregelkammer rasch den Vorgaben des plethysmographischen Messsignals folgen kann.

Sobald die Rückkopplungsschleife geschlossen wird, ist allerdings der Arbeitspunkt bzw. der Setpoint nicht mehr optimal nachkontrollierbar. Deswegen muss bei bekannten Verfahren zumindest Intermittierend die kontinuierliche Messung für eine Nachjustierung des Setpoints unterbrochen werden, bzw. kann es während der Messung zu einem Verlust des Setpoints kommen, wodurch ein Blutdruckabfall oder Anstieg vortäuscht werden kann. Deswegen war es für den Anwender bisher nicht ersichtlich ob eine beobachtete Änderung des Blutdrucks oder der Blutdruckamplitude durch eine physiologische oder pathologische Änderung des intraarteriellen Blutdrucks oder aber durch einen Verschiebung des Setpoints entstanden ist. Damit ist das Verfahren derzeit für die Intensivstation oder den Operationssaal, wo es am meisten benötigt würde, nicht einsetzbar.

Aus der DE 38 29 456 A1 ist ein Blutdruckmessgerät mit zwei Oberarmmanschetten bekannt, die beide knapp unter dem diastolischen Blutdruck bei gering unterschiedlichen Drücken arbeiten. Gemäß einer Ausführungsvariante können die beiden Manschetten als Doppelfingermanschette ausgebildet sein. Die Druckmessung erfolgt allerdings nicht nach der oben beschriebenen, bevorzugten "vascular unloading technique".

In der DE 39 35 939 A1 wird ein nicht-invasives Blutdruckmessgerät beschrieben, das eine optischen Wandler zur Gewinnung eines Blutdrucksignals aufweist, wobei eine konventionelle elektronische Blutdruckmanschette zur Kalibrierung des Blutdruckmessgerät vorgesehen ist.

Aus der EP 0 377 554 A1 ist ein Verfahren zur Messung des Blutdruckes bekannt, bei welchem der Blutdruck an wenigstens einer Messstelle und einer Referenz-Messstelle gemessen wird. Die Phasenverschiebung des zwischen der Messstelle und der Referenz-Messstelle aufgezeichneten Blutdrucksignals wird zur Berechnung des Blutdruckes herangezogen.

Aufgabe der Erfindung ist es, eingangs beschriebene Vorrichtungen zur kontinuierlichen, nicht-invasiven Messung des Blutdruckes nach der "vascular unloading technique" derart zu verbessern, dass eindeutig festgestellt werden kann, ob eine beobachtete Änderung des Blutdrucks oder der Blutdruckamplitude auf eine physiologische oder pathologische Änderung des intraarteriellen. Blutdrucks oder aber auf eine Verschiebung des Arbeitspunktes rückzuführen ist.

Diese Aufgabe wird erfindungsgemäß dadurch gelöst, dass die Druckmesskammer der zweiten Druckmanschette als eine simultan und unabhängig von der Druckmesskammer der ersten Druckmanschette regelbare Referenzdruckkammer ausgebildet ist, dass die Druckmesskammer der ersten Druckmanschette und die Referenzdruckkammer der zweiten Druckmanschette jeweils separate Einlass- und Auslassventile aufweisen, wobei der Druck in der Referenzdruckkammer über die Regel- und Steuereinrichtung nach einer vorgebbaren Druckfunktion regelbar ist. Es kann somit gleichzeitig ein Druckmesssignal und ein Referenzsignal gewonnen werden. Die zweite Druckmesskammer wird unabhängig von der ersten Druckmesskammer als Referenzdruckkammer betrieben wobei der Druck in der Referenzdruckkammer gemäß einer vorgebbaren Druckfunktion geregelt und zeitlich simultan zum Druckmesssignal ein Referenzsignal gewonnen wird und das Referenzsignal zur Interpretation des Druckmesssignals herangezogen wird.

Die vorliegende Erfindung vermeidet somit die eingangs erwähnten Nachteile durch Schaffung der Möglichkeit der gleichzeitigen und kontinuierlichen Überwachung der closed-loop Blutdruckmessung durch Schaffung einer "Watchdog" Einrichtung, nämlich der open loop Messung in der Referenzdruckkammer. Dazu müssen ähnlich oder ident dimensionierte bzw, ausgestaltete Druckkammern über Arterien ähnlicher bzw. identer Dimension angebracht werden, Z.B. hervorragend geeignet hierfür ist die in der WO 00/59369 A2 beschriebene Doppelfingermanschette, welche allerdings erst im Sinne der vorliegenden Erfindung adaptiert werden muss, unter Anderem derart, dass die Druckmesskammer der ersten Druckmanschette und die Referenzdruckkammer der zweiten Druckmanschette jeweils separate Einlass- und Auslassventile aufweisen müssen.

Obwohl die Druckmesskammer und die Referenzdruckkammer über den verschiedensten Arterien, z.B. auch über Arteria temporalis und Arteria radialis angeordnet sein können, ist es - insbesondere für Langzeltmesstungen - von Vorteil, die beiden Druckmanschetten an benachbarten Arterien, vorzugsweise an zwei benachbarten Fingern einer Hand, anzubringen. Die andere Hand steht dann für notwendige, intensivmedizinische Zugänge frei.

Das Referenzsignal in der Referenzdruckkammer kann mit Hilfe eines dort angeordneten Drucksensors oszillometrisch oder vorzugsweise plethysmographisch gemessen werden, wenn gemäß zweier Varianten der Erfindung die zweite Druckmanschette eine oszillometrische Sensoreinrichtung oder bevorzugt eine zweite plethysmographische Sensoreinrichtung aufweist. Wenn in der Referenzdruckkammer kein plethysmographischer Sensor angeordnet ist, kann In der Referenzdruckkammer nur die Höhe der durch den Arterienpuls hervorgerufenen Druckoszillationen bei verschiedenen beaufschlagten Drucken gemessen werden, wie dies aus der oszillatorischen Blutdruckmessung bekannt ist. Das Maximum der Oszillationen entspricht dabei dem arteriellen Mitteldruck.

Die im Wesentlichen identen Druckkammern und Lichtmessstrecken der plethysmographischen Sensoreinrichtungen der beiden Manschetten haben erfindungsgemäß gleichzeitig eine gegenteilige Funktion. Während die plethysmographischen Sensoreinrichtung der Druckmesskammer auf "closed-loop" geschaltet ist und den Druck In der Kammer so regelt, dass das Lichtsignal konstant und der transmurale Druck $PT_m$ gleich oder in etwa Null ist, ist die jeweils andere Druckkammer auf open loop Betrieb ("Watchdog") geschaltet.

Der Druck in der Referenzdruckkammer kann gemäß einer sich wiederholenden Stufen- oder Rampenfunktion geregelt werden. Intermittierend wird die Referenzdruckkammer mit Druckänderungen, z.B. Drucktreppen oder Druckrampen beaufschlagt, um den Arbeitspunkt bzw. Setpoint ständig zu kontrollieren oder nachzuregeln bzw. um zwischen Änderungen des Setpoints und tatsächlichem physiologischem oder pathologischem Driften des arteriellen Blutdrucks zu unterscheiden.

Gemäß einer besonders vorteilhaften Weiterbildung sind zumindest zwei verschiedene Überwachungszustände vorgesehen: Einer, bei der die Änderung des plethysmographischen Referenzsignals nach Beaufschlagung der Referenzdruckkammer mit einer vorgebbaren Druckfunktion, z.B. Drucktreppen, nur beobachtet wird (open-loop) und ein anderer Überwachlungszustand, bei dem der Druck in der Referenzdruckkammer gemäß der vorgebbaren Druckfunktion und gleichzeitig mit Hilfe des plethysmographisch erfassten Referenzsignals derart geregelt wird, dass die Amplitude des Referenzsignals minimiert wird, wobei ein Referenzdrucksignal gemessen wird (semi-closed-loop). Dabei wird versucht das plethysmographische Signal auch während der Druckänderung z.B, während der Drucktreppe zu minimieren. Der erste der beiden Zustände der Referenzdruckkammer wird in der Folge auch open-loop-Treppe, der zweite auch semi-closed-loop-Treppe genannt, während der Zustand der Druckmesskammer als closed-loop bezeichnet wird. Mit Hilfe dieser Anordnung kann der Arbeitspunkt der closed-loop Druckmesskammer ständig durch die Watchdog- bzw. Referenzdruckkammer nachgeregelt werden, bzw. zwischen echten Blutdruckveränderungen Im Körper und einem Shift des Setpoints absolut verlässlich unterschieden werden.

Bei einer Änderung des Mitteldruckes und/oder der Amplitude des Druckmesssignals und einer fehlenden oder gegensinnigen Verschiebung des Amplitudenmaximums des Referenzsignals bzw. des Referenzdrucksignals kann auf einen Verlust des Arbeitspunktes des Drucksignals geschlossen werden.

Durch den völlig gleichartigen Aufbau der Druckkammern der beiden Druckmanschetten kann in vorgebbaren Zeitintervallen oder bei einem Verlust des Arbeitspunktes die Referenzdruckkammer als Druckmesskammer und die Druckmesskammer als Referenzdruckkammer betrieben werden. Es kann somit jederzeit die Watchdog-Druckkammer zur closed-loop Druckmesskammer umgeschaltet werden und umgekehrt. Diese Anordnung hat auch den großen Vorteil, dass der Körperteil, z.B. der Finger, der für die closed-loop Messung verwendet wird, immer wieder automatisch oder manuell gewechselt werden kann, ohne dass die registrierte Pulskurve unterbrochen wird. Dazu muss unmittelbar vor der Umschaltung lediglich die letzte Ermittlung des Setpoints in der Referenzdruckkammer verwendet werden, um den Setpoint für die closed-loop Messung zu eruieren und um dann auf eine closed-loop Messung des Blutdrucks auf diesem Finger umzuschalten, bevor die closed-loop Messung am anderen Finger beendet wird. Der andere Finger wird in der Folge zum Auffindung und Überwachung des Setpoints benützt.

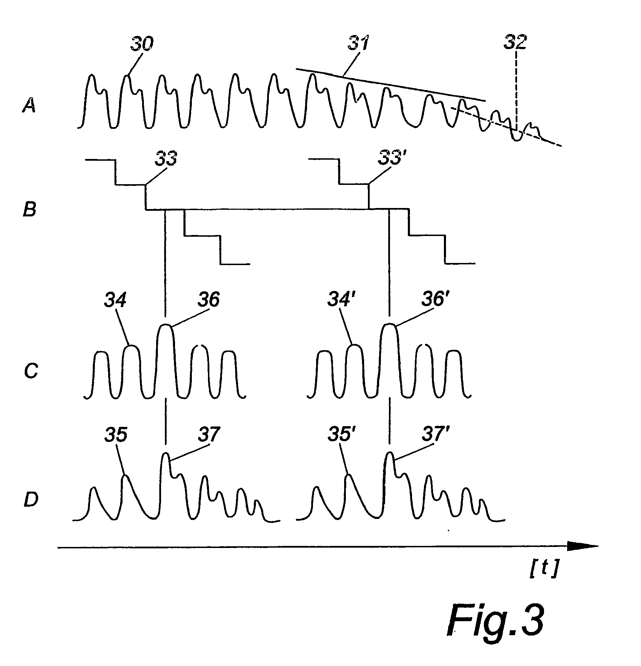

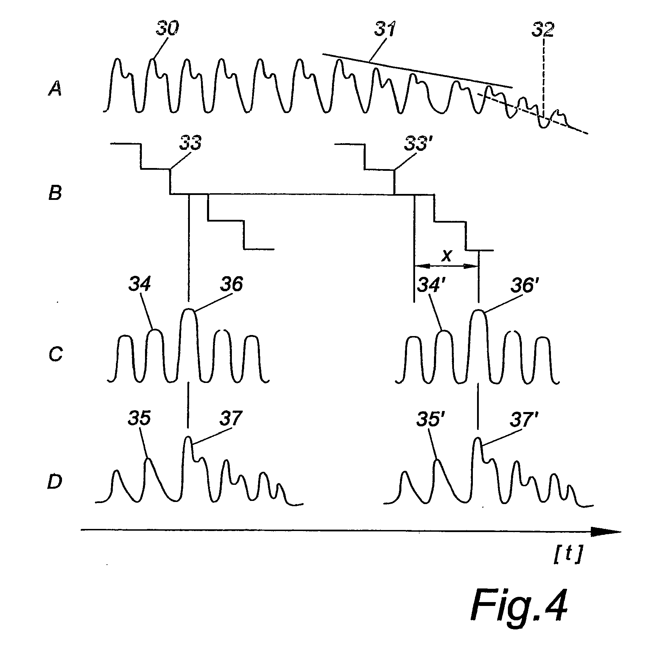

Aus einer Änderung des Mitteldruckes und/oder der Amplitude des Druckmesssignals und einer gleichsinnigen Verschiebung des Amplitudenmaximums des Referenzsignals bzw, des Referenzdrucksignals kann auf eine physiologische oder pathologische Änderung des Druckmesssignals geschlossen werden. Details dazu werden anhand der Diagramme der Fig. 3 und Fig. 4 näher erläutert.

Das bei verschiedenen, vorgebbaren Druckwerten der Druckfunktion gemessene Referenzdrucksignal kann analysiert, mit vorgegebenen, idealen Pulskurven verglichen und bei minimaler Abweichung von einer vorgegebenen Pulskurve daraus der Arbeitspunkt für das Druckmesssignal bestimmt werden.

Vor der Ausgabe der Druckkurve sollte durch die Regel- und Steuereinrichtung, welche vorzugsweise als Mikroprozessor ausgeführt ist, die Blutdruck-Folgekurve auf die vorausgegangene Blutdruckkurve sowohl hinsichtlich absoluter Höhe als auch hinsichtlich der Amplitude justiert werden. Dies sollte allerdings in vorteilhafter Weise nur bei geplanter, routinemäßiger Umschaltung, nicht jedoch bei vorher stattgefundenen nicht korrigierten Verschiebungen des Setpoint erfolgen. Mit der neuen Vorrichtung wird erstens die Druckbelastung und Blutstauung in den beiden Fingern entscheidend reduziert, zweitens erfolgt die Messung einer Druckkurve ohne jede Unterbrechung und echte Druckänderungen im Körper können erstmals online von Änderungen des Setpoints mit absoluter Sicherheit unterschieden werden. Damit entstehen derart große Vorteile, dass erstmals diese nicht-invasive Messung der kontinuierlichen Pulskurve für die Patientenüberwachung auf Intensivstationen oder im Operationssaal einsetzbar wird.

Die Erfindung wird im Folgenden anhand von Zeichnungen näher erläutert. Es zeigen:

Fig. 1 in schematischer Darstellung eine erfindungsgemäße Vorrichtung zur kontinuierlichen, nicht-Invasiven Messung des Blutdruckes nach dem Prinzip der entspannten Arterienwand,

Fig. 2 Details einer Ausführungsvariante der Vorrichtung gemäß Fig. 1,

Fig. 3 bis Fig. 5 Diagramme mit Mess- bzw. Drucksignalen der erfindungsgemäßen Messvorrichtung,

Fig. 6 eine Details einer weiteren Ausführungsvariante in einer schematischen Darstellung, sowie

Fig. 7 ein Diagramm zur Ausführungsvariante gemäß Fig. 6.

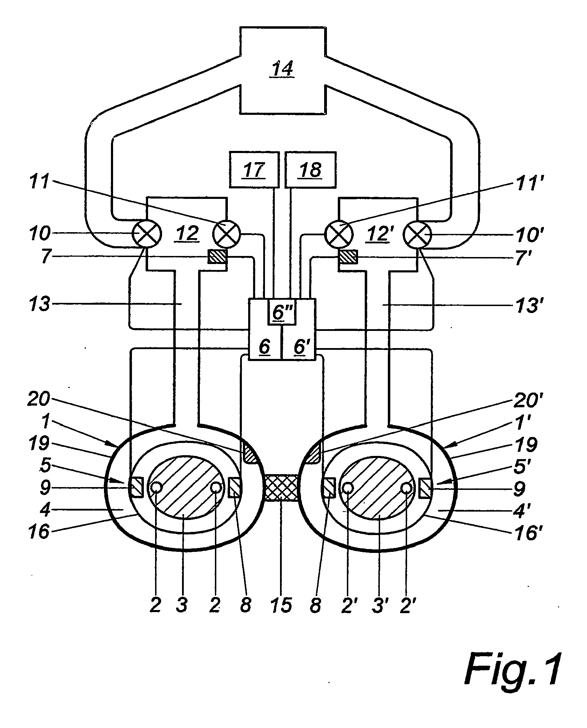

Fig. 1 zeigt eine Vorrichtung zur kontinuierlichen, nicht-invasiven Messung des Blutdruckes, mit zumindest einer ersten Druckmanschette 1, welche an einem ersten, eine Arterie 2 enthaltenden Körperteil oder Körperbereich 3 anbringbar ist und eine aufblasbare Druckmesskammer 4, sowie eine erste plethysmographische Sensoreinrichtung 5 aufweist, wobei eine Regel- und Steuereinrichtung 6 vorgesehen ist, welche den Druck in der Druckmesskammer 4 mit Hilfe des Messsignals der plethysmographischen Sensoreinrichtung 5 derart regelt, dass die Amplitude des plethysmographischen Messsignals minimiert wird. Die Druckmesskammer 4 steht mit zumindest einem Drucksensor 7 zur Gewinnung eines Druckmesssignals in Verbindung. Die erfindungsgemäße Vorrichtung weist eine zweite Druckmanschette 1' auf, welche an einem zweiten, eine Arterie 2' enthaltenden Körperteil oder Körperbereich 3' anbringbar ist und eine mit der Druckmesskammer 4 baugleiche, aufblasbare Referenzdruckkammer 4' (Watchdog-Druckkammer) mit einem Drucksensor 7' aufweist. Der Druck in der Referenzdruckkammer 4' wird über die Regel- und Steuereinrichtung 6 nach einer vorgebbaren Druckfunktion geregelt. Die zweite Druckmanschette 1'.weist eine zweite plethysmographische Sensoreinrichtung 5' auf.

Im dargestellten Beispiel welsen die plethysmographischen Sensoreinrichtungen 5, 5' jeweils Uchtemitter 8 und Lichtdetektoren 9 auf und können somit die pulsatilen Änderungen des durchstrahlten Volumens detektieren. Die separaten Einlass- 10, 10' und Auslassventile 11, 11' der Druckmesskammer 4 und der Referenzdruckkammer 4' sind in separaten Druckregelkammern 12, 12' angeordnet, welche jeweils über Druckleitungen 13, 13' mit der Druckmesskammer 4 und der Referenzdruckkammer 4', sowie über die Einlassventile 10, 10' mit einer gemeinsamen Druckquelle 14 in Verbindung stehen. Es wäre auch möglich, die Einlass- 10, 10' und Auslassventile 11, 11' sowie die Drucksensoren 7, 7' direkt in der Druckmesskammer 4 bzw. In der Referenzdruckkammer 4' anzuordnen, wobei die Druckregelkammern 12, 12' entfallen könnten.

Die beiden Druckmanschetten 1, 1' sind bevorzugt ringförmig ausgebildet und In Form einer Doppelfingermanschette im Wesentlichen starr über das Verbindungselement 15 miteinander verbunden. Die Druckkammern 4, 4' sind innen jeweils mit einer leicht verformbaren Membran 16, 16' ausgestattet. Die starre Verbindung 15 hat den Vorteil, dass die zwischen der Druckkammer 4, 4' und dem jeweiligen Finger 3, 3' angebrachten Lichtemitter 8 und Lichtempfänger 9 der plethysmographischen Sensoreinrichtungen 5, 5' in ihrer Lage konstant und in optimaler Position zur jewelligen Arterie 2, 2' zu liegen kommen. Damit müssen vor Messbeginn lediglich die beiden ringförmigen Druckkammern 4, 4' der Doppelfingermanschette auf die Finger 3, 3' aufgeschoben werden wodurch die richtige Lage der Lichtemitter 8 und Lichtdetektoren 9 garantiert ist. Die separaten Ein- und Auslassventile 10, 11 bzw. 10', 11' der beiden Druckregelkammern 12, 12' werden von der beispielsweise durch einen oder mehrere Mikroprozessoren 6, 6', 6" realisierten Regel- und Steuereinrichtung angesteuert.

Die Regel und Steuereinrichtung bewältigt gleichzeitig unterschiedliche Aufgaben. So können entweder unterschiedliche Prozessoren 6 bzw. 6' gleichzeitig unterschiedliche Regelaufgaben bewerkstelligen oder es kann ein multi-tasking bzw. multi-threading fähiger Prozessor 6, 6' vorhanden sein. Eine übergeordnete Einheit 6" koordiniert die Aufgaben bzw. übernimmt Anzeigen und Alarme. So können die Druckmesskammer 4 und die Referenzdruckkammer 4' mit einem unterschiedlichen Druck bei gleicher Druckquelle 14 versorgt werden. Die Regel- und Steuereinrichtung bedient auch eine Anzeige- 17 und Alarmeinrichtung 18. Zur Verringerung der Compliance der beiden Druckkammern 4, 4' ist es vorteilhaft, wenn diese auf der dem Körperteil abgewandten, äußeren Seite eine relativ starre Wand 19 aufweisen. In diese, die Compliance vermindernde, starre Wand oder von dieser ausgehend Ist jeweils zumindest ein Temperaturfühler 20, 20' ein- oder angebaut.

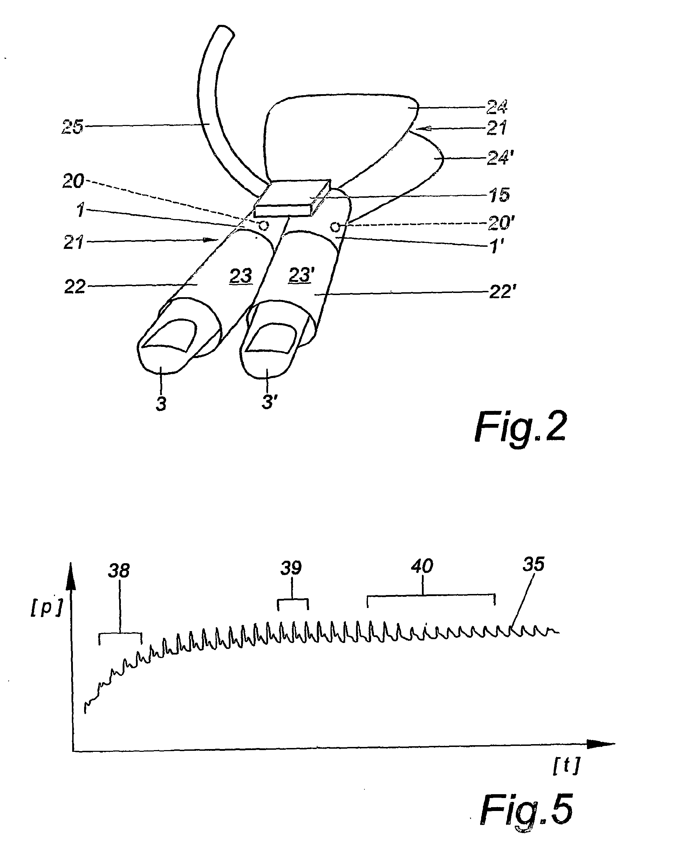

Gemäß der in Fig. 2 dargestellten Ausführungsvariante ist eine Heizeinrichtung 21 in die beiden Druckmanschetten 1, 1' integriert oder an diese anschließend ausgebildet, welche zumindest ein Heizelement 22, 22', vorzugsweise eine Heizfolie oder eine Heizspirale aufweist. Die Heizeinrichtung 21 kann auch auf die Doppelfingermanschette aufsteckbar ausgeführt sein. Damit kann die Druckkammer bzw. der Körperteil, dessen Arterie zur Untersuchung herangezogen wird, z.B. der Finger 3 bzw. 3', so erwärmt werden, dass es auch bei einer Zentralisierung des Kreislaufes, wie z.B. bei Schock, nicht zu einem Verlust des Volums- oder Flusssignals der beiden pletysmographischen Sensoreinrichtungen 5, 5' in den beiden Druckkammern der Druckmanschetten 1, 1' kommt. Neben den physiologischen Temperaturen von ca. 37 Grad kann man sich hier auch das Prinzip der durch Überwärmung induzierten Hyperämie zu Nutze machen.

Die Heizeinrichtung 21 kann heizbare Fortsätze, beispielsweise Fingerlinge 23, 23' aufweisen, welche sich distal in Richtung Körperperipherie erstrecken. Diese Fingerlinge können sich z.B. bis zur Fingerspitze oder nahe zu dieser fortsetzen. Die Heizeinrichtung 21 kann weiters sich proximal in Richtung Körperzentrum erstreckende Fortsätze 24, 24' aufweisen, welche beispielsweise an der Innenhand und am Handrücken anliegen Die Heizeinrichtung kann auch fäustlingartig über mehrere Finger oder flächenartig ausgebildet sein und beispielsweise mit einem Klettverschluss um die Hand geschlossen und befestigt werden. Günstig auf Intensivstationen ist es jedoch, wenn zumindest die Fingerkuppen frei bleiben, weil diese dann vom Arzt zur Beurteilung der Durchblutung oder Sauerstoffsättigung herangezogen werden können

Es ist von Vorteil ein gemeinsames Kabel 25 vorzusehen, welches pneumatische und elektrische Anschlüsse für die beiden Druckmanschetten 1, 1' sowie für die Heizeinrichtung 21 enthält, da gerade auf Intensivstationen oder im Operationssaal jede zusätzliche Leitung den klinischen Betrieb sehr erschwert. Die Heizeinrichtung 21 kann zumindest einen In einer der Druckmanschetten 1, 1' angeordneten Temperaturfühler 20, 20' aufweisen, dessen Temperatursignal zur Regelung der Heizleistung der Heizeinrichtung 21 dient.

Die in den Fig. 3 und Fig. 4 dargestellten Diagramme A, B, C und D weisen dieselbe Zeitachse t auf, wobei auf den Ordinaten der Diagramme A, B und D Druckwerte und auf der Ordinate des Diagramms C die Intensität des pletysmographischen Referenzsignals der Referenzdruckkammer aufgetragen ist. Bei der Verwendung von zwei Druckkammern, einer Druckmesskammer 4 und einer Referenzdruckkammer 4' In jeweils einer Druckmanschette, können die Diagramme C und D nur alternativ beobachtet werden, da die oben beschriebenen, unterschiedlichen Funktionszustände der Referenzdruckkammer (Watchdog-Druckkammer), nämlich open loop Zustand (Diagramm C) und semi-closed-loop Zustand (Diagramm D), nur nacheinander dargestellt werden können. Bei der Verwendung von einer Druckmesskammer und zwei Referenzdruckkammern (Dreifingermanschette) können beide Zustände und somit die Diagramme C und D gleichzeitig beobachtet werden. Etwalge Ungenauigkeiten der Kurvensynchronizität in den Fig. 3 und Fig. 4 sind nur zeichnerisch bedingt. Alle Kurven in dieser Abbildung sind auch um einen allfälligen "Base Line Drift" bereinigt.

Dabei zeigt Fig. 3 Im Diagramm A das im Betrieb in der closed-loop-Druckmesskammer 4 beobachtete typische Druckmesssignal 30 mit einer Änderung der Druckamplitude 31 und des Mitteldruckes 32 des Blutdrucks verursacht durch ein Driften des Arbeitspunktes bzw. Setpoints.

Im Diagramm B ist beispielhaft eine vorgebbare Druckfunktion 33, 33', z.B. die Druckstufen einer konsekutiven Stufenfunktion, dargestellt, mit welcher die Watchdog-Druckkammer 4' beaufschlagt wird. Im Diagramm C ist dabei das in der Watchdog-Druckkammer 4' beobachtete plethysmographische Referenzsignal 34, 34', z.B. das Signal des Lichtdetektors, der pletysmographischen Sensoreinrichtung 5' gezeigt und alternativ dazu im Diagramm D das Referenzdrucksignal 35, 35' in der Watchdog-Druckkammer 4' bei Beaufschlagung mit der Stufenfunktion 33, 33' jedoch nun bei gleichzeitig minimierter Amplitude des Referenzsignals 34, 34' (sogenannte semi-closed-loop-Treppe). Während Diagramm C die Volumsamplltuden im open loop darstellt, sind im Diagramm D die Druckamplituden im semi-closed-loop bei Minimierung der Volumssignalschwankungen und zusätzlich beaufschlagten verschiedenen Drucken gemäß Stufenfunktion dargestellt. Wie im Diagramm C ersichtlich, zeigen die von der Watchdog-Druckkammer registrierten Amplitudenschwankungen des Referenzsignals 34 eine zeitlich, idente Lokalisation der Amplituden sowie des Amplitudenmaximums 36, 36' in Relation zu den jeweils beaufschlagten identen Drucken der Stufenfunktion 33, 33'. Das Amplitudenmaximum 37, 37' im Diagramm D zeigt ebenfalls eine idente Lokalisation bei identen Drucken in den konsekutiv beaufschlagten Stufenfunktion 33, 33'.

Die beobachtete Diskrepanz, nämlich der sich ändernden Druckamplitude 31 und des sich ändernden Mitteldruckes 32 in der closed-loop-Druckmesskammer einerseits (Diagramm A), und auf der anderen Seite idente oder ähnliche Lokalisation der Amplituden sowie des Amplitudenmaximums 36, 36' des Referenzsignals 34 (Diagramm C) bei den gleichen Druckstufen der Stufenfunktion 33, 33' (Diagramm B) in der Watchdog-Druckkammer, bzw. idente oder ähnliche Lokalisation der Druckamplituden 37, 37' des Referenzdrucksignals 35, 35' in Relation zu den Druckstufen der Stufenfunktion 33, 33' In der semi-closed-loop Funktion, zeigen einen Verlust des Arbeitspunktes bzw. des Setpoints an und deuten nicht auf einen echten Blutdruckabfall hin, Es kann nun entweder automatisch die Watchdog-Druckkammer 4' zur closecl-loop-Druckmesskammer 4 umgeschaltet werden und umgekehrt, oder der Setpoint der closed-loop-Druckmesskammer nachjustiert werden.

Auch bei einer - im Hinblick auf die In der closed-loop-Druckmesskammer gemessenen Druckänderung - gegensinnigen Verschiebung des Amplitudenmaximums 36, 36' im Diagramm C bzw. des Maximums der Druckamplitude 37, 37' im Diagramm D (z.B. Verschiebung der maximalen Amplitude 36, 36' des Referenzsignals 34 (open loop) und Druckamplitude 37, 37' (semi-closed-loop) In der Watchdog-Druckkammer zu höheren Drucken bei gleichzeitigem Absinken des Mitteldruckes 32 oder der Blutdruckamplitude 31 im Druckmessignal 30 zu niederen Drucken), sollte eine Umschaltung der Watchdog-Druckkammer zur Druckmesskammer bzw. eine Korrektur des Setpoints in der Druckmesskammer erfolgen.

Fig. 4 zeigt Im Diagramm A der Abbildung dieselbe Änderung der Druckamplitude 31 und des Mitteldrucks 32 des Druckmesssignals 30 wie In Fig. 3 dargestellt, hier verursacht hingegen durch eine tatsächliche pathologische Veränderung des Blutdrucks. Dies ist sofort aus der Verschiebung X der maximalen Amplitude 36, 36' des Referenzsignals 34, 34' (Diagramm C, in open loop Funktion) und alternativ aus den maximalen Druckamplituden 37, 37' des Referenzdrucksignals 35, 35' (Diagramm D, in semi-closed-loop Funktion) gemessen In der Watchdog-Druckkammer erkennbar, denn es tritt bei konsekutiven gleichen Drucktreppen der Stufenfunktion 33, 33' (Diagramm B) eine Verschiebung X der Lokalisation der Amplitudenmaxima in den Diagrammen C und D auf. Diagramm D zeigt - wie in Fig. 3 - die Druckamplituden im semi-closed-loop bei Minimierung der Volumssignalschwankungen und zusätzlich beaufschlagten, verschiedenen Drucken gemäß Stufenfunktion. Im gegenständlichen Fall findet sich die maximale Amplitude 36' des Referenzsignals 34' (Diagramm C, open loop ) und alternativ die maximale Druckamplitude 37' (Diagramm D, semi-closed-loop) bei deutlich geringeren Blutdruckwerten. Dies weist eindeutig auf einen echten Blutdruckabfall hin und wäre sofort ein Grund einen optischen und/oder akustischen Alarm auf der Alarmeinrichtung 18 auszulösen, wenn gewisse Grenzwerte über- oder unterschritten werden. Damit ist die gegenständliche Vorrichtung zur kontinuierlichen Blutdruckmessung überwachungstauglich und genügt den höchsten Ansprüchen.

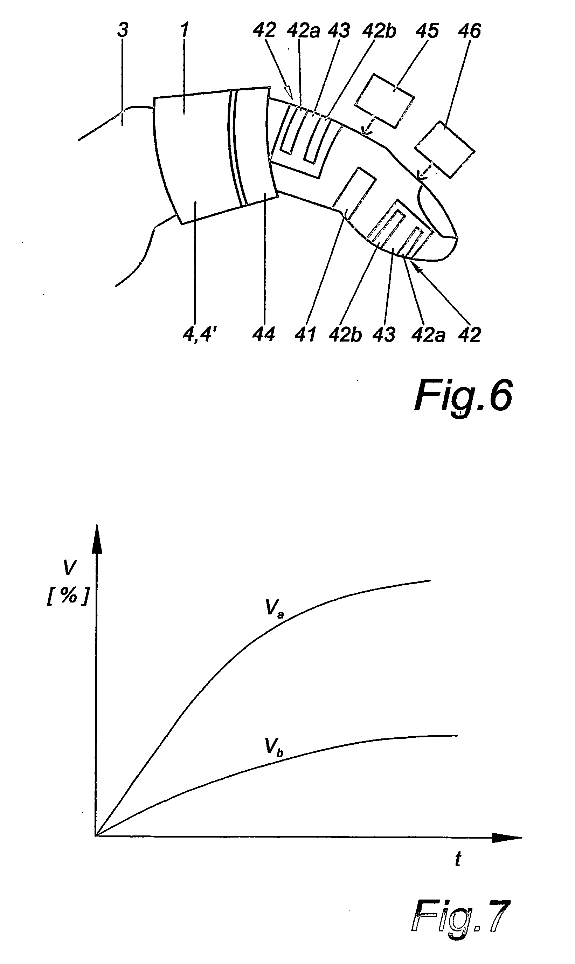

Wie in Fig. 5 dargestellt, ist neben der Lokalisation der Druckamplitude auch die Kurvenform In den Bereichen 38, 39 und 40 des Referenzdrucksignals 35 im semi-closed-loop, gemessen in der Referenzdruckkammer bzw. der Watchdog-Druckkammer bei Beaufschlagung mit Drucktreppen, charakteristisch dafür, ob sich der Arbeitspunkt bzw. der Setpoint verschoben hat, oder nicht. Wie aus Fig. 5 ersichtlich ändert sich die Kurvenform der Pulswelle, abhängig davon, ob der durch die Drucktreppen im semi-closed-loop beaufschlagte Druck bei gleichzeitiger Minimierung des Referenzsignals zu niedrig 38, optimal 39 oder zu hoch 40 ist. Bei optimalem Druck in der Watchdog-Druckkammer entspricht die Druckkurve genau der physiologischen Druckkurve, wie sie aus invasiven Messungen bekannt ist, nämlich mit einem Stellanstieg, einem runden Gipfel, einer dikroten Welle in typischer Höhe der Hälfte bis rund zwei Drittel der Druckamplitude und einem annähernd exponentiellem diastolischen Abfall der Pulskurve wie bei 39 gezeigt. Hingegen, bei zu niedrigem Druck der Drucktreppe, verliert die Pulskurve in der semi-closed-loop nicht nur an Amplitude, sie verliert ebenfalls ihre physiologische Form, sie wird breit und flach wie bei 38 gezeigt, außerdem wandert die dikrote Inzisur Richtung diastolischem Blutdruck. Ist der Druck der Drucktreppe im semi-closed-loop zu hoch 40, wird die Pulskurve sehr spitz und die dikrote Welle entspricht nicht mehr der physiologischen Pulsform 39.

Das heißt, dass neben der Amplitude der Druckwelle auch die Form der Pulswelle herangezogen werden kann, um den optimalen Druck der Drucktreppe im semi-closed-loop zu ermitteln. Dazu muss nur eine Formanalyse der Kurve durchgeführt werden und deren Abweichung von einer vorgegebenen idealen Pulskurve ermittelt werden. Bei dem Druck der Drucktreppe im semi-closed-loop, bei dem die Abweichungen der ermittelten Pulskurve von einer vorgegebenen Pulskurve am kleinsten sind, entspricht der Gegendruck dem idealen Gegendruck mit Annäherung des transmuralen Druckes $P_{Tm}$ gegen Null und daher dem Setpoint der in der Druckmesskammer eingestellt werden sollte. Es ist bekannt, dass sich z.B. durch das Lebensalter oder durch Atherosklerose die physiologische Pulsform ändert. So wandert z.B. die dikrote Welle mit steiferen Gefäßen Richtung systo-Ilschem Gipfel und kann z.B. auch in diesem gänzlich verschwenden, daher kann es günstig sein, nicht nur eine, sondern mehrere physiologische Druckkurven im Mikroprozessor zu speichern, mit denen dann die ermittelte Pulskurve im semi-closed-loop verglichen wird.

Bei den beaufschlagten Druckschwankungen In der Referenzdruckkammer bzw. der Watchdog-Druckkammer muss es sich natürlich nicht unbedingt um eine Treppe oder Rampe handeln, auch jede andere Form einer vorgebbaren Druckfunktion wäre anwendbar und im Sinne der Anmeldung zu nützen.

Die beschriebenen Ausführungsvarianten sind nur beispielhafte Ausführungen der Vorrichtung gemäß Erfindung, denkbar wären viele andere Ausprägungen, wie z.B. auch Druckkalotten anstatt der hier gezeigten Druckmanschetten, die z.B. auch über der Arteria radialis oder Arterie temporalis angebracht werden könnten. Denkbar wären z.B. auch voneinander disloziierte Druckkammern an anderen Extremitäten, die aber den Auswerteaufwand erschweren, da nur in derselben Extremität und in gleich oder sehr ähnlich dimensionierten Arterien idente Druck und Flussverhältnisse des Blutes herrschen. Auch kann neben den beschriebenen optischen pietysmographischen Sensoreinrichtungen jede andere Art der Fluss- oder Volumsmessung herangezogen werden.

Weiters ist vorgesehen, dass die Druckmessung in den kleinen Arterien, z.B. den Fingerarterien, an den Druck in einer großen Arterie, der unabhängig gemessen wird, rechnerisch angepasst wird, da bekannt ist, dass der Druck in den kleinen Arterien nicht unbedingt dem Druck in den großen Arterien entspricht. Dafür muss lediglich zumindest am Anfang oder auch Intermittierend der Druck in einer großen Arterie mit einer unabhängigen Vorrichtung gemessen werden und dann die kontinuierliche Messung des Blutdruckes wie In der gegenwärtigen Erfindung beschrieben, sowohl bezüglich des systolischen Blutdruckes als auch des diastolischen Blutdruckes im Absolutwert dem Druck wie an der großen Arterie gemessen, angepasst werden.

Falls notwendig, kann, z.B. mit einer Flüssigkeitssäule, der hydrostatische Druck zwischen der Druckmesskammer und/oder der Referenzdruckkammer (Watchdog-Druckkammer) einerseits und der Höhe des Herzens gemessen, und die ermittelte Druckkurve wie in den Diagrammen A der Fig. 3 und Fig. 4 gezeigt, um diesen hydrostatischen Druckunterschied zwischen der Druckmesskammer 4 und/oder Watchdog-Druckkammer 4' bereinigt werden. Dies ist besonders dann wichtig, wenn der Körperteil, am dem die beiden Druckkammern 4, 4' bzw. deren Druckmanschetten 1, 1' befestigt sind, sich laufend in seiner relativen Höhe dem Herzen gegenüber verlagert.

Weiters ist zu beachten, dass ein mit Druck beaufschlagte Körperteil, z.B. der Finger 3, durch die kontinuierlich und rhythmisch aufgepumpte Druckmesskammer 4 in einer unkontrollierten Weise unphysiologisch belastet wird. Weiters kann - trotz vorhandener "Watchdog Funktion" der Referenzdruckkammer - der Arbeitspunkt der Druckmesskammer verloren gehen, weil durch die partielle Okklusion durch die Messdruckkammer im untersuchten Körperteil andere physiologische Adaptationen ablaufen als im Körperteil der durch die Referenzdruckkammer beaufschlagt wird.

Die damit zusammenhängenden Probleme haben eine gemeinsame Ursache, nämlich die, dass der Blutfluss im untersuchten Körperteil, z.B. dem Finger, bereits rein physiologisch um einen Faktor 100 schwanken kann. Diese großen Schwankungen des absoluten Blutflusses bewirken die Abweichung vom Blutdruck in kleinen Arterien zum Druck in den großen Arterien. Ein geringer Blutfluss in der Peripherie der Zirkulation, z.B.

im Finger ist die Folge einer Engsteilung der Arteriolen, damit entsteht an diesen Engstellen eine starke Reflexion der Pulswelle und damit eine Überhöhung des Druckes in kleinen Arterien gegenüber dem Druck in der vorgeschalteten großen Arterie; umgekehrt führen offene Arteriolen zu einer geringeren Engstellung, einer geringeren Reflexion und damit zu einer geringeren oder fehlenden Überhöhung oder gar zu einer Erniedrigung des Druckes gegenüber den vorgeschalteten großen Arterien. Wenn die Volumsänderung bzw. der absolute oder relative Blutfluss in der Peripherie, z.B. im Finger bekannt ist, Ist auch das Ausmaß der Reflexion der Druckwelle in den der Druckmesskammer nachgeschalteten Arteriolen bekannt und damit kann der In der Peripherie gemessene Blutdruckwert auf den absoluten Wert in den großen Arterien korrigiert werden.

Erfindungsgemäß kann daher - wie in Fig. 6 dargestellt - distal von der Druckmesskammer 4 und/oder der Referenzdruckkammer 4' zumindest ein Sensor 41, 42, 44 zur Messung einer Volumsänderung des Körperteils 3 vorgesehen sein. Beispielsweise kann am Körperteil 3, vorzugsweise am distalen Ende des Fingers, ein Impedanzsensor 42, Dehnmessstreifen 41 und/oder ein weiterer plethysmographischer Sensor 44 angeordnet sein. Dabei wird die Änderung des Volumens des Körperteils 3 bevorzugt bei einem Druck der Druckmesskammer und/oder Referenzdruckkammer von kleiner als dem arteriellen Blutdruck, z.B. bei ca. 40 mm Hg gemessen. Die Änderung des Volumens des Körperteils distal von der Druckmesskammer und/oder Referenzdruckkammer können dann zur rechnerischen Korrektur des von der Druckmesskammer laufend ermittelten Blutdrucks verwendet werden.

Die Messgenauigkeit kann zusätzlich erhöht werden, wenn distal von der Druckmesskammer 4 und/oder der Referenzdruckkammer 4' zumindest ein Sensor 45 zur Messung des Blutflusses, beispielsweise ein Venenverschlussplethysmograph oder Laser-Doppler-Blutflussmessgerät, vorgesehen ist.

Wie in Fig. 6 schematisch angedeutet, können am Körperteil 3 beispielsweise am untersuchten Finger zumindest ein Dehnmessstreifen 41 - oder mehrere Impedanzelektroden 42 - angebracht werden, die den Volumsstatusides untersuchten Körperteils distal der Druckmanschetten 1 und der darin angeordneten Messkammer 4 laufend überwachen. Bei den Impedanzelektroden ist zu beachten, dass die jeweils äußeren Elektroden 42a der Stromeinspeisung dienen, während die jeweils innere Elektrode 42b der Impedanzmessung dient. Je eine Strom- und eine Impedanzelektrode könnte dabei z.B. auf einer gemeinsamen Trägerfolie 43 befestigt sein. Bei Erhöhung des Druckes In der Druckmesskammer 4 und/oder Referenzdruckkammer 4' auf z.B. 40 mm Hg fließt anfänglich nur Blut in den untersuchten Körperteil 3 hinein aber keines mehr heraus: Dies bedeutet eine Volumszunahme V des Körperteils mit der Zeit t, wie im Diagramm gemäß Fig. 7 gezeigt. Ein steiler Anstieg der Volumszunahme bedeutet hohen Blutfluss

(Volumskurve $V_a$) und damit eine andere Korrektur der Blutdruckwerte als diese bei einem niedrigen Blutfluss (Volumskurve $V_b$) zu erfolgen hat. Geringer Blutfluss und langsame Volumszunahme resultieren aus einer Kontraktion der Arteriolen und bedeuten eine stärkere Reflexion der Pulswelle nach zentral und damit höhere Blutdruckwerte am untersuchten Körperteil als bei niedrigem Fluss und geringer Volumszunahme des Körperteils. Die Korrektur der Blutdruckwerte zur Bestimmung des Interessierenden Blutdrucks in den großen Arterien erfolgt dabei am besten empirisch durch Ermitteln der Abweichung des in der Druckmesskammer 4 ermittelten Blutdruckes von einem gleichzeitig mit einem anderen konventionellen Verfahren gemessenen Blutdruckes an einer großen Arterie in Abhängigkeit von der prozentuellen Volumszunahme des Körperteils distal der Druckmesskammer und/oder der Referenzdruckkammer während der Venenokklusion.

Von Vorteil ist die Kenntnis der Volumszunahme und damit des Blutflusses beispielsweise In untersuchten Finger, da bei großer Volumszunahme ein rascher Wechsel von Druckmesskammer und Referenzdruckkammer durchgeführt werden kann, um einer Überfüllung und damit einem Ödem des untersuchten Körpertelles vorzubeugen. Bei niedrigem Blutfluss genügt hingegen ein größeres Intervall bis zum Wechsel von Druckmesskammer und Referenzdruckkammer.

Weiters ist kann eine kritische Volumszunahme des Fingers auch Ursache des Verlusts des Arbeitspunktes bei der Blutdruckmessung durch die Druckmesskammer sein. Diese kritische Volumszunahme kann ebenfalls durch die Volumsmessung des Fingers z.B. mit einem Venenverschlussplethysmographen erkannt werden Erst findet sich nämlich eine rasche initiale Volumszunahme, solange das Blut ungehemmt einströmen kann, bei Congestion des Fingers kommt es zum Ansteigen des Gewebeinnendruckes, daher findet sich dann später eine geringere Volumszunahme, damit aber auch eine Kompression der Arterien und Abnahme des Volumssignals und damit Verlust des Arbeitspunktes der Druckmesskammer. Dem kann mit der Volumsmessung distal der Druckmesskammer und/oder Referenzdruckkammer vorgebeugt werden, indem bei kritischen Veränderungen der Füllungskurve des untersuchten Körperteils Druckmesskammer 4 und/oder Referenzdruckkammer 4' rechtzeitig gewechselt werden.

Gemäß einer Weiterbildung der Erfindung kann distal von der Druckmesskammer 4 und/oder der Referenzdruckkammer 4' zumindest ein Sensor 46 zur Messung von Blutgasen, beispielsweise des $CO_2$ oder des $O_2$ Partialdruckes vorgesehen sein. Die Messung des Sauerstoffdruckes oder der $CO_2$-Konzentration im Gewebe kann zur Regelung des Druckes In der Druckmesskammer 4 und/oder der Referenzdruckkammer verwendet werden.

Die Messung des Sauerstoffdruckes oder des Kohledioxydruckes distal der Druckmesskammer und/oder Referenzdruckkammer beispielsweise durch die bekannten transkutanen Sauerstoff- oder Kohledioxyd-messgeräte kann als Warnvorrichtung für den notwendigen Wechsel bzw. zur Steuerung der Druckmesskammer und/oder Referenzdruckkammer von Vorteil sein, das ein Absinken des Partialdruckes von Sauerstoff und oder ein Anstieg des Kohlendioxyddruckes distal der Druckmesskammer und/oder Referenzdruckkammer ein Anschwellen des Gewebes und damit einen drohenden Verlust des Arbeitspunktes anzeigen kann.

Die Messung des Blutflusses beispielsweise am Finger hat auch noch weitere klinische Vorteile: ein Blutdruckabfall, z.B. auch ein lebensbedrohender Schockzustand, kann nämlich einerseits durch Zentralisation des Kreislaufs, z.B. durch Blutverlust, entstehen, was gleichzeitig mit einer Abnahme des Blutflusses in der Peripherie, also auch im Finger, einhergeht. Auf der anderen Seite kann ein Schock auch durch eine zu große Öffnung in peripheren Gefäßen entstehen, so dass das Füllungsvolumen des Kreislaufs nicht mehr ausreicht, um den Blutdruck aufrecht zu halten, wie das z.B. Im septischen Schock der Fall ist. In diesem Fall ist der Blutfluss in der Peripherie hoch. Die erste Form des Schocks benötigt eine ganz andere Therapie als die zweite. Somit kann mit der Vorrichtung gemäß der Erfindung auch eine Differentialdiagnose von Blutdruckabfällen also auch von Schockzuständen durchgeführt werden, die dann eine bessere Therapie ermöglicht.

## Patentansprüche

1. Vorrichtung zur kontinuierlichen, nicht-invasiven Messung des Blutdruckes nach dem Prinzip der entspannten Arterienwand, mit zumindest einer ersten (1) und einer zweiten Druckmanschette (1') vergleichbarer oder identer Dimension, welche an einem ersten und an einem zweiten jeweils eine Arterie (2, 2') vergleichbarer oder identer Dimension enthaltenden Körperteil oder Körperbereich (3, 3') anbringbar sind und jeweils eine aufblasbare Druckmesskammer (4. 4') aufweisen, wobei die erste Druckmanschette (1) eine erste plethysmographische Sensoreinrichtung (5) aufweist, die mit einer Regel- und Steuereinrichtung (6) verbunden ist, welche den Druck in der ersten Druckmesskammer (4) mit Hilfe des Messsignals der plethysmographischen Sensoreinrichtung (5) regelt, und wobei die Druckmesskammer (4) mit zumindest einem Drucksensor (7) zur Gewinnung eines Druckmesssignals in Verbindung steht, **dadurch gekennzeichnet, dass** die Druckmesskammer der zweiten Druckmanschette (1') als eine simultan und unabhängig von der Druckmesskammer (4) der ersten Druckmanschette regelbare Referenzdruckkammer (4') ausgebildet ist, dass die Druckmesskammer (4) der ersten Druckmanschette (1) und die Referenzdruckkammer (4') der zweiten Druckmanschette (1') jeweils separate Einlass-(10, 10') und Auslassventile (11, 11') aufweisen, wobei der Druck in der Referenzdruckkammer (4') über die Regel- und Steuereinrichtung (6) nach einer vorgebbaren Druckfunktion regelbar ist.

2. Vorrichtung nach Anspruch 1, **dadurch gekennzeichnet, dass** die zweite Druckmanschette (1') eine zweite plethysmographische Sensoreinrichtung (5') aufweist.

3. Vorrichtung nach Anspruch 1 oder 2, **dadurch gekennzeichnet, dass** die separaten Einlass (10, 10')- und Auslassventile (11, 11') der Druckmesskammer (4) und der Referenzdruckkammer (4') in separaten Druckregelkammern (12, 12') angeordnet sind, welche jeweils über separate Druckleitungen (13, 13') mit der Druckmesskammer (4) und der Referenzdruckkammer (4'), sowie über die Einlassventile (10, 10') mit einer gemeinsamen Druckquelle (14) in Verbindung stehen.

4. Vorrichtung nach einem der Ansprüche 1 bis 3, **dadurch gekennzeichnet, dass** eine Heizeinrichtung (21) in die Druckmanschetten (1, 1') integriert oder an diese anschließend ausgebildet ist, welche zumindest ein Helzelement (22, 22'), vorzugsweise eine Heizfolie oder eine Heizspirale, aufweist.

5. Vorrichtung nach einem der Ansprüche 1 bis 4, **dadurch gekennzeichnet, dass** distal von der Druckmesskammer (4) und/oder der Referenzdruckkammer (4') zumindest ein Sensor (41, 42, 44) zur Messung einer Volumsänderung des Körperteils (3, 3') vorgesehen ist.

6. Vorrichtung nach Anspruch 5, **dadurch gekennzeichnet, dass** am Körperteil (3), vorzugsweise am distalen Ende des Fingers, ein Impedanzsensor (42), Dehnmessstreifen (41) und/oder ein weiterer plethysmographischer Sensor (44) vorgesehen ist.

7. Vorrichtung nach einem der Ansprüche 1 bis 6, **dadurch gekennzeichnet, dass** die Referenzdruckkammer (4') als Druckmesskammer (4) und die Druckmesskammer (4) als Referenzdruckkammer (4') betreibbar ist.

8. Vorrichtung nach einem der Ansprüche 1 bis 7, **dadurch gekennzeichnet, dass** die beiden Druckmanschetten (1, 1') an benachbarten Arterien, vorzugsweise an zwei benachbarten Fingern (3, 3') einer Hand, anbringbar sind.

## Claims

1. A device for the continuous, non-invasive measurement of blood pressure based on the principle of the relaxed arterial wall, with at least one first (1) and one second pressure cuff (1') of identical or comparable size, which can be attached on at least one first and one second body part or body region (3, 3') containing an artery (2, 2') of identical or comparable size, each having an inflatable pressure measuring chamber (4, 4'), the first pressure cuff (1) being provided with a first plethys-mographic sensor device (5) connected to a controlling and adjusting device (6), which controls the pressure in the first pressure measuring chamber (4) using the measuring signal of the plethysmographic sensor device (5), and where the pressure measuring chamber (4) is connected to a pressure sensor (7) in order to obtain a pressure measuring signal, **characterised in that** the pressure measuring chamber of the second pressure cuff (1') is configured as a reference pressure chamber (4'), which is controlled simultaneously with and independently of the pressure measuring chamber (4) of the first pressure cuff, and that the pressure measuring chamber (4) of the first pressure cuff (1) and the reference pressure chamber (4') of the second pressure cuff (1') each have separate inlet valves (10, 10') and outlet valves (11, 11'), with the pressure in the reference pressure chamber (4') being controlled via the controlling and adjusting device (6) in accordance with a preselectable pressure function.

2. Device according to claim 1, **characterised in that** the second pressure cuff (1') is provided with a second plethysmographic sensor device (5').

3. Device according to claims 1 or 2, **characterised in that** the separate inlet (10, 10') and outlet (11, 11') valves of the pressure measuring chamber (4) and the reference pressure chamber (4') are placed in separate pressure control chambers (12, 12'), which are each connected by separate pressure lines (13, 13') to the pressure measuring chamber (4) and the reference pressure chamber (4') and via the inlet valves (10, 10') to a common pressure source (14).

4. Device according to any of claims 1 to 3, **characterised in that** a heating unit (21) is integrated in or appended to the two pressure cuffs (1, 1'), which is provided with at least one heating element (22, 22'), preferably a heating foil or a heating spiral.

5. Device according to any of claims 1 to 4, **characterised in that** at least one sensor (41, 42, 44) is provided at a location distal to the pressure measuring chamber (4) and/or the reference pressure chamber (4') for measuring a volume change of the body part (3,3').

6. Device according to claim 5, **characterised in that** an impedance sensor (42), strain gauges (41) and/or an additional plethysmographic sensor (44) is positioned on the body part (3), preferably at the distal end of the finger.

7. Device according to any of claims 1 to 6, **characterised in that** the reference pressure chamber (4') can be operated as pressure measuring chamber (4) and the pressure measuring chamber (4) as reference pressure chamber (4').

8. Device according to any of claims 1 to 7, **characterised in that** the two pressure cuffs (1, 1') are positioned on two neighbouring arteries, preferably on two adjacent fingers (3, 3') of one hand.

**Revendications**

1. Dispositif pour la mesure non invasive continue de la tension artérielle suivant le principe du relâchement de la paroi artérielle, comportant au moins un premier manchon (1) et un second manchon (1') de dimensions comparables ou identiques qui peuvent s'appliquer à une première et à une seconde partie du corps ou zone du corps (3, 3') renfermant chacune une artère (2, 2') de dimensions comparables ou identiques, et qui présentent chacun une chambre de mesure de la tension (4, 4') gonflable,

le premier manchon (1) présentant un premier dispositif de détection pléthysmographique (5) raccordé à un dispositif de régulation et de commande (6) qui règle la pression de la première chambre de mesure de la tension (4) à l'aide du signal de mesure issu du dispositif de détection pléthysmographique (5), et

la chambre de mesure de la tension (4) étant raccordée à au moins un capteur de tension (7) permettant de produire un signal de mesure de la tension, **caractérisé en ce que**

la chambre de mesure de la tension du second manchon (1') se présente sous la forme d'une chambre de pression de référence (4') réglable simultanément et indépendamment de la chambre de mesure de la tension (4) du premier manchon, la chambre de mesure de la tension (4) du premier manchon (1) et la chambre de pression de référence (4') du second manchon (1'), présentant chacune des soupapes d'entrée (10, 10') et de sortie (11, 11') séparées, et la pression régnant dans la chambre de pression de référence (4') est réglable au moyen du dispositif de réglage et de commande (6) suivant une fonction de pression prédéterminable.

2. Dispositif selon la revendication 1, **caractérisé en ce que** le second manchon (1') présente un second dispositif de détection pléthysmographique (5').

3. Dispositif selon la revendication 1 ou 2,
**caractérisé en ce que**
les soupapes d'entrée (10, 10') et de sortie (11, 11')
séparées de la chambre de mesure de la tension (4)
et de la chambre de pression de référence (4') sont
installées dans des chambres de régulation de pression (12, 12') séparées qui communiquent chacune
avec la chambre de mesure de la tension (4) et la
chambre de pression de référence (4') par l'intermédiaire de conduites de pression (13, 13') séparées
et avec une source de pression commune (14) par
l'intermédiaire des soupapes d'entrée (10, 10').

4. Dispositif selon l'une des revendications 1 à 3,
**caractérisé en ce qu'**
un dispositif de chauffage (21) est intégré aux manchons (1, 1') ou est adjacent à ceux-ci en présentant
au moins un élément de chauffage (22, 22'), de préférence une feuille chauffante ou une spirale chauffante.

5. Dispositif selon l'une des revendications 1 à 4,
**caractérisé par**
au moins un capteur (41, 42, 44) éloigné de la chambre de mesure de la tension (4) et/ou de la chambre
de pression de référence (4') pour mesurer une modification volumétrique de la partie du corps (3, 3').

6. Dispositif selon la revendication 5,
**caractérisé par**
un capteur d'impédance (42), une jauge extensométrique (41) et/ou un autre capteur pléthysmographique (44) sur la partie du corps (3) et de préférence
sur l'extrémité distale du doigt.

7. Dispositif selon l'une des revendications 1 à 6,
**caractérisé par**
la chambre de pression de référence (4') peut faire
fonction de chambre de mesure de la tension (4) et
la chambre de mesure de la tension (4) peut faire
fonction de chambre de pression de référence (4').

8. Dispositif selon l'une des revendications 1 à 7,
**caractérisé en ce que**
les deux manchons (1, 1') peuvent s'appliquer à des
artères voisines, de préférence à deux doigts voisins
(3, 3') d'une main.

*Fig.1*

**Fig.2**

**Fig.5**

*Fig.3*

*Fig.4*

Fig.6

Fig.7